(19) Europäisches Patentamt
European Patent Office
Office européen des brevets

(11) Publication number: 0 219 896 B1

(12) # EUROPEAN PATENT SPECIFICATION

(45) Date of publication of patent specification: **03.11.93**

(51) Int. Cl.[5]: **A61K 31/485**, A61K 9/06, A61K 9/12

(21) Application number: **86201655.7**

(22) Date of filing: **24.09.86**

(54) **Pharmaceutical compositions on the basis of dextrorphan for intranasal application.**

(30) Priority: **26.09.85 IT 2228885**

(43) Date of publication of application:
**29.04.87 Bulletin 87/18**

(45) Publication of the grant of the patent:
**03.11.93 Bulletin 93/44**

(84) Designated Contracting States:
**AT BE CH DE FR GB LI LU NL SE**

(56) References cited:
**EP-A- 0 131 315**
**GB-A- 1 121 358**
**US-A- 4 454 140**

**The J. of Pharmacology and Experimental Therapeutics, vol. 120, p. 528, 532 (1957)**

(73) Proprietor: **PRODOTTI FORMENTI S.r.l.**
**Via Correggio 43**
**I-20149 Milano(IT)**

(72) Inventor: **Del Soldato, Piero**
**Via Toti, 22**
**I-20052 Monza(IT)**
Inventor: **Casadio, Silvano**
**Via Tantardini, 15**
**I-20136 Milan(IT)**

(74) Representative: **Appoloni, Romano et al**
**Ing. Barzanò & Zanardo S.p.A.**
**Via Borgonuovo 10**
**I-20121 Milano (IT)**

EP 0 219 896 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

Dextrorphan is the dextro isomer of levorphan, this latter being an opium substance endowed with marked analgesic activity. Differently from levorphan, dextrorphan does not have any analgesic activity, nor has it any affinity for the opium receptors (and therefore it causes neither dependence nor tolerance) (Benson W.M., Stefko P.L., Randall L.O.; J . Pharmacol. Exp. Therap. 109, 189, 1953), but is endowed with a considerable antitussis action (Goodman L.S., Gilman A.: "The Pharmacological Basis of Therapeutics", page 263, McMillan Pub. Co. Inc., New York 1975).

Many investigations have shown that this compound, either in free or in conjugated form, is the major metabolite of dextromethorphan (Axelrod J., J. Pharmacol. Exp. Ther. 117, 322, 1956; Elison C ., Elliott, J., Pharmacol. Exp. Ther. 144, 265, 1964; Kamm J., Taddeo A., Van Loon E., J . Pharmacol. Exp. Ther. 158, 437, 1967; Willner, Arzneim. Forsch. 13, 20, 1963), a drug which has been marketed worldwide as a sedative for tussis. It has been demonstrated indeed (Aylward M., Maddock J., Davies D.E., Protheroe D. A., Leideman T.; Eur. J. Respir. Dis. 65, 283, 1984) that the clinical efficaciousness of dextromethorphan is best correlated not to its hematic levels (which are practically negligible in correspondence of the peak time of the therapeutical effects), but to the hematic levels of dextrorphan, so that the concentration of said metabolite in the biological fluids is commonly determined for the kinetic study of dextromethorphan (Ramachander G., Williams F.D., Emele J.F.; J. Pharm. Sci. 66, 1047, 1977). The hematic levels of dextrorphan which are observed after the oral administering of dextromethorphan remain stable over longer than three hours. But it must be remembered that the metabolic conversion of dextromethorphan into dextrorphan does not occur always in a constant and foreseeable way. In fact, this reaction depends on sex (Ramachander G., Bapatla K.R., Emele J.F.; J. Pharm. Sci. 67, 1326, 1978), i.e., it is much faster in male sex, and, like all the metabolic reactions of the exogenous substances, it can be influenced by the state of health of the patient, by his age, etc. (Goodman L.S., Gilman A.: "The Pharmacological Basis of Therapeutics", pages 16-17, Mc Millan Int. Co., New York 1975).

Another interesting consideration is that dextrorphan has a toxicity by parenteral way even lower than dextromethorphan (Benson W.M., Stefko P.L., Randall L.O.; J. Pharmacol. Exp. Therap., 109, 189, 1953). One could thus think to directly use dextrorphan as a tussis sedative agent.

But a severe hindrance to a therapeutical use thereof is constituted by the fact that this component is not absorbed when it is administered by oral way (Schanker L.S., Shore Parkhurst P.A., Brodie B.B., Hoghen C.A.M., J. Pharmacol. Exp. Therap., 120, 528, 1957).

To obviate this drawback, the present invention proposes to use for dextrorphan the intranasal administering way, which allows the product to come, integer and in substantial amount, to the effector organ. According to the invention it has been observed indeed that the intranasal administration of pharmaceutical compositions containing dextrorphan has as its effect a high bioavailability of the active principle. These results lead to an excellent therapeutical performance, comparable to that deriving from the parenteral administering.

Surprisingly, it has been found according to the invention that dextrorphan can be efficaciously adminstered by intranasal way, in suitable pharmaceutical compositions.

More specifically, the inventors found that salified dextrorphan can be advantageously formulated in new dosage forms for intranasal administration, to the purpose of producing an improved bioavailability and homogeneity of its hematic levels, relatively to the oral formulation. The new forms of administering of the present invention can be solutions, suspensions, ointments, gels, nebulized aerosols, fitted to the intranasal application. Thus, for an aspect thereof, the present invention supplies a pharmaceutically acceptable nasal-release composition, in a form of unitary dosage, for the intranasal administering, to the purpose of obtaining an antitussive effect in warm-blooded animals:

I) by the use of a therapeutically efficacious amount of active principle, substantially dextrorphan salts, as better specified in the descriptive section of the invention;

II) non-toxic and pharmaceutically acceptable nasal carriers.

For another aspect thereof, the present invention relates to compositions for intranasal application of pharmaceutical quality, in form of unitary dosage, for intranasal administering, having as their purpose the obtaining of a systemic therapeutical response:

I) in warm-blooded animals by the use of salified dextrorphan - which shall be better specified in the detailed description of the invention - used as antitussis agent;

II) by the use of non-toxic and pharmaceutically valid nasal carriers, viz., pharmaceutical compositions comprising nasal ointments, nasal gels and pressurized aerosols.

For still another aspect thereof, the present invention supplies a system to obtain a systemic therapeutical response in warm-blooded animals in the therapy which uses antitussis drugs, by resorting to

the use of dextrorphan salts, better specified in the detailed disclosure of the invention, administered by intranasal way, in combination with non-toxic and pharmaceutically valid nasal carriers.

The compound selected for the use in the compositions and in the systems of the present invention is dextrorphan, which can be represented by the following structural formula:

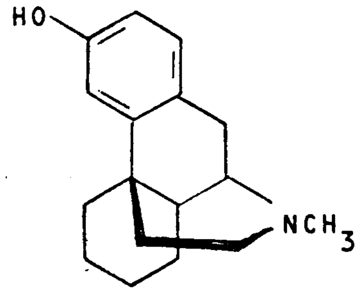

The compound can be salified by a whatever acid, e.g., tartaric acid.

According to the present invention, dextrorphan, as a salt (e.g., as the tartrate), can be administered by intranasal way with considerably higher results than those obtainable after the oral administration, in terms of increased bioavailability of the active principle, and minimization of the variations in hematic levels.

It appears well evident that the above mentioned compound is rapidly absorbed by the nasal mucosa into the systemic hematic circulation without first-pass metabolism. The above mentioned compound can be conveniently administered by intranasal way to warm-blooded animals by means of formulations suitable to be administered by intranasal application, formulations which comprise the dextrorphan salt in the proper amount to perform the antitussis effect, together with a pharmaceutically acceptable and non-toxic carrier.

The choice of the traditionally acceptable and non-toxic carriers, which does not exclude the use of carriers traditionally mentioned in the art of nasal administration, depends on the chemical-physical characteristics of the dextrorphan salt, on the required posology, on the type of selected formulation (solution, nasal gel, nasal ointment, and spray aerosol,.), on the stability of the same salt. The preferred dosage forms by intranasal way are the solutions, creams, and the gels which contain a major water amount besides the active principle. Minor amounts of other ingredients, such as preservatives, buffering agents, wetting agents and gelifiers can be sometimes present. This type of composition can be used in the management of tussis.

Compatibly with the nature and the complexity of the formulation, it is preferable that the formulation be isotonic.

The carriers on prevailingly aqueous and low-viscosity basis, as well as those using propellants on the basis of halocarbons, tend to increase the absorption rate.

The carriers based on gelified matrices, on Oil/Water (O/W) or W/O emulsions, used for direct intranasal application or by atomization, allow effects more prolonged over time to be obtained, without penalizing the fastness of these effects to any significant extent.

Of course, the therapeutical dosage range for the nasal administration of dextrorphan, according to the present invention, shall vary with the weight of the patient, and with the patient's condition the drug is administered for. The typical dosage for dextrorphan could be comprised within the range of from 10 to 100 mg, for administering by nasal way twice or three times per day.

Of course, the amount of form of nasal dosage to release the desired dosis shall depend on the concentration of the drug in the composition. For example, the volume of solution or of gel necessary to release dextrorphan in the above detailed doses shall range from 0.1 to 1 .0 g of solution and/or cream or salve or gel at 10%.

Examples of the preparation of typical nasal compositions containing dextrorphan tartrate are reported hereunder.

## EXAMPLES

### Example 1

| | |
|---|---|
| Dextrorphan tartrate | 10 g |
| Methyl p.hydroxybenzoate | 0.08 g |
| Propyl p.hydroxybenzoate | 0.02 g |
| Glycerol | 20 g |
| Purified water | q.s. to 100 ml |

Dissolve dextrorphan tartrate in an aliquot of water; dissolve parabens in glycerol and add this solution to the previous one. Bring to full volume.

### Example 2

| | |
|---|---|
| Dextrorphan tartrate | 10 g |
| Hydroxypropylmethylcellulose | 0.5 g |
| Methyl p.hydroxybenzoate | 0.08 g |
| Propyl p.hydroxybenzoate | 0.02 g |
| Glycerol | 10 g |
| Purified water | q.s. to 100 ml |

Dissolve parabens in glycerol.

In 70% of water, dissolve at 40°C dextrorphan tartrate and hydroxypropylmethylcellulose. At room temperature, add the two solutions to each other. Bring to full volume with water.

Example 3

NASAL GEL

| | | |
|---|---|---|
| Dextrorphan tartrate | 15 | g |
| Carboxypolymethylene | 1 | g |
| Propylene glycol | 20 | g |
| Methyl p.hydroxybenzoate | 0.08 | g |
| Propyl p.hydroxybenzoate | 0.02 | g |
| Triethanolamine | 1.10 | g |
| Purified water | q.s. to 100 | ml |

In an aliquot of water, dissolved is dextrorphan tartrate and added is carboxypolymethylene.

Parabens are dissolved in propylene glycol; the solution obtained is added to the previous one.

By using the residual amount of water, dissolve triethanolamine; add this solution to the previous one, with careful mixing and stirring.

The gel is applied as a normal ointment for nasal use.

Example 4

EMULSION

| | | |
|---|---|---|
| Dextrorphan tartrate | 15 | g |
| Cetylstearyl alcohol | 18 | g |
| Esters of a polyol and fatty acids | 9 | g |
| Distilled water | 55 | g |

Dextrorphan tartrate is dissolved at 80 °C in 55 g of distilled water. Cetylstearyl alcohol and the esters of a polyol and fatty acids are melted at 80 °C with stirring in a suitable equipment.

To this mixture, the previously prepared solution is added with stirring at 80 °C. Stirring is continued at room temperature, until a homogeneous mixture is obtained.

Example 5

**SALVE**

| | |
|---|---|
| Dextrorphan tartrate | 15 g |
| Carboxypolymethylene | 1 g |
| Triethanolamine | 1.25 g |
| Methyl p.hydroxybenzoate | 0.08 g |
| Propyl p.hydroxybenzoate | 0.02 g |
| Liquid paraffin | 10 g |
| Vaseline oil | 5 g |
| Castor oil | 5 g |
| Purified water | q.s. to 100 g |

Dextrorphan tartrate, and then, with stirring, carboxypolymethylene is dissolved in an aliquot of water. The solution is then added of triethanolamine in the residual amount of water: the blend is carefully mixed and is heated to 65 °C.

Separately, a solution has been obtained by heating to 65 °C liquid paraffin, vaseline oil and castor oil.

The oily solution is added to the aqueous solution, and stirring is continued, while their temperature is slowly decreased to room value.

The ointment is applied as a normal ointment for nasal use.

Example 6

| | |
|---|---|
| Dextrorphan tartrate | 10 g |
| Polyglycol esters of fatty acids | 20 g |
| Distilled water | q.s. to 100 g |

Dextrorphan tartrate is dissolved at 70 °C in distilled water. The polyglycolic ester of fatty acids is melted at 65 °C with stirring in a suitable vessel. To this compound, added is, with stirring, and at 65 °C, the previously prepared solution.

The whole mass is stirred at room temperature, until a homogeneous mixture is obtained.

Example 7

| | |
|---|---|
| Dextrorphan tartrate | 15 g |
| Liquid paraffin | 8 g |
| Lanolin O.P. | 3 g |
| White Vaseline | q.s. to 100 g |

Melt the excipients, add dextrorphan tartrate, homogenize and cool to room temperature.

Example 8

PRESSURIZED AEROSOL

| | |
|---|---|
| Dextrorphan tartrate | 10 g |
| Soybean lecithin | 0.6 g |
| Ethanol | 5.5 g |
| FREON® | 20 g |
| FREON® | 70.9 g |

Dextrorphan tartrate, previously pulverized, is dispersed in about 10 ml of absolute ethanol; soybean lecithin and the propellants are added, and the whole is conditioned inside aerosol cans.

**Claims**
**Claims for the following Contracting States : BE, CH, DE, FR, GB, LI, LU, NL, SE**

**1.** Pharmaceutically acceptable composition suitable to achieve a systemic therapeutic response to antitussis in warm-blooded animals, comprising: (I) a therapeutically efficacious amount of salified dextrorphan at a concentration between 10% and 15%, and (II) a pharmaceutically acceptable non-toxic excipient for intranasal administration per dosage unit.

**2.** Pharmaceutical composition as defined in claim 1, characterized in that it is a nasal suspension.

**3.** Pharmaceutical composition as defined in claim 1, characterized in that it is a nasal salve.

**4.** Pharmaceutical composition as defined in claim 1, characterized in that it is a nasal cream.

**5.** Pharmaceutical composition as defined in claim 1, characterized in that it is a nasal solution.

**6.** Pharmaceutical composition as defined in claim 1, characterized in that it is a nasal gel.

**7.** Pharmaceutical composition as defined in claim 1, characterized in that it is a long-release gel.

**8.** Pharmaceutical composition as defined in claim 1, characterized in that it is nasal drops.

**9.** Pharmaceutical composition as defined in claim 1, characterized in that it is a nasal spray.

**10.** Composition as defined in claim 1, wherein (I) is an amount therapeutically efficacious by systemic way due to the pharmacological action of salified dextrorphan.

**Claims for the following Contracting State : AT**

**1.** Process for preparing a pharmaceutically acceptable composition suitable to achieve a systemic therapeutic response to antitussis in warm-blooded animals, characterised by admixing (I) a therapeutically efficacious amount of salified dextrorphan at a concentration between 10% and 15%, and (II) a pharmaceutically acceptable non-toxic excipient for intranasal administration per dosage unit.

**2.** Process for preparing a pharmaceutical composition as defined in claim 1, characterized in that it is a nasal suspension.

**3.** Process for preparing a pharmaceutical composition as defined in claim 1, characterized in that it is a nasal salve.

**4.** Process for preparing a pharmaceutical composition as defined in claim 1, characterized in that it is a nasal cream.

**5.** Process for preparing a pharmaceutical composition as defined in claim 1, characterised in that it is a nasal solution.

**6.** Process for preparing a pharmaceutical composition as defined in claim 1, characterised in that it is a nasal gel.

**7.** Process for preparing a pharmaceutical composition as defined in claim 1, characterized in that it is a long-release gel.

**8.** Process for preparing a pharmaceutical composition as defined in claim 1, characterised in that it is nasal drops.

**9.** Process for preparing a pharmaceutical composition as defined in claim 1, characterized in that it is a nasal spray.

**10.** Process for preparing a composition as defined in claim 1, wherein (I) is an amount therapeutically efficacious by systemic way due to the pharmacological action of salified dextrorphan.

**Patentansprüche**
**Patentansprüche für folgende Vertragsstaaten : BE, CH, DE, FR, GB, LI, LU, NL, SE**

**1.** Pharmazeutisch verträgliche Zusammensetzung, geeignet, um eine körpereigene, therapeutische Reaktion auf Hustenreiz lindernde Mittel bei warmblütigen Lebewesen zu erreichen, die (I) eine therapeutisch wirksame Menge von in Salzform vorliegendem Dextrorphan bei einer Konzentration zwischen 10% und 15% und (II) einen pharmazeutisch verträglichen, nicht toxischen Arzneimittelträger für die intranasale Verabreichung pro Dosierungseinheit umfaßt.

**2.** Pharmazeutische Zusammensetzung nach Anspruch 1,
dadurch **gekennzeichnet,** daß
sie eine nasale Suspension ist.

**3.** Pharmazeutische Zusammensetzung nach Anspruch 1,
dadurch **gekennzeichnet,** daß
sie eine nasale Salbe ist.

**4.** Pharmazeutische Zusammensetzung nach Anspruch 1,
dadurch **gekennzeichnet,** daß
sie eine nasale Creme ist.

**5.** Pharmazeutische Zusammensetzung nach Anspruch 1,
dadurch **gekennzeichnet,** daß
sie eine nasale Lösung ist.

**6.** Pharmazeutische Zusammensetzung nach Anspruch 1,
dadurch **gekennzeichnet,** daß
sie ein nasales Gel ist.

**7.** Pharmazeutische Zusammensetzung nach Anspruch 1,
dadurch **gekennzeichnet,** daß
sie ein langzeitfreisetzendes Gel ist.

**8.** Pharmazeutische Zusammensetzung nach Anspruch 1,
dadurch **gekennzeichnet,** daß
sie in Form von nasalen Tropfen vorliegt.

**9.** Pharmazeutische Zusammensetzung nach Anspruch 1,
dadurch **gekennzeichnet,** daß
sie ein nasales Spray ist.

**10.** Zusammensetzung nach Anspruch 1,
dadurch **gekennzeichnet,** daß
(I) eine hinsichtlich der körpereigenen Weise therapeutisch wirksame Menge, bedingt durch die pharmakologische Wirkung des in Salzform vorliegenden Dextrorphans, ist.

**Patentansprüche für folgenden Vertragsstaat : AT**

**1.** Verfahren zur Herstellung einer
pharmazeutisch verträglichen Zusammsetzung, geeignet, um eine körpereigene, therapeutische Reaktion auf Hustenreiz lindernde Mittel bei warmblütigen Lebewesen zu erreichen, dadurch **gekannzeichnet,** daß man (I) eine therapeutisch wirksame Menge von in Salzform vorliegendem Dextrorphan bei einer Konzentration zwischen 10 % und 15 % und (II) einen pharmazeutisch verträglichen, nichttoxischen Arzneimittelträger für die intranasale Verabreichung pro Dosierrnngseinheit miteinander vermengt.

**2.** Verfahren zur Herstellung einer
pharmazeutischen Zusammensetzung nach Anspruch 1, dadurch **gekennzeichnet,** daß die Zusamensetzung eine nasale Suspension ist.

**3.** Verfahren zur Herstellung einer
pharmazeutischen Zusammensetzung nach Anspruch 1, dadurch **gekennzeichnet,** daß die Zusammensetzung eine nasale Salbe ist.

**4.** Verfahren aur Herstellung einer
pharmazeutischen Zusammensetzung nach Anspruch 1, dadurch **gekennzeichnet,** daß die Zusammensetzung eine nasale Creme ist.

**5.** Verfahren zur, Herstellung einer
pharmazeutischen, Zusammensetzung nach Anspruch 1, dadurch **gekennzeichnet,** daß die Zusammensetzung eine nasale Lösung ist.

**6.** Verfahren zur Herstellung einer
pharmazeutischen Zusammensetzung nach Anspruch 1, dadurch **gekennzeichnet,** daß die Zusammensetzung ein nasales Gel ist.

**7.** Verfahren zur Herstellung einer
pharmazeutischen Zusammensetzung nach Anspruch 1, dadurch **gekennzeichnet,** daß die Zusammensetzung ein langzeitfreisetzendes Gel ist.

**8.** Verfahren zur Herstellung einer
pharmazeutischen Zusammensetzung nach Anspruch 1, dadurch **gekennzeichnet,** daß die Zusammensetzung in Form von nasalen Tropfen vorliegt.

**9.** Verfahren zur Herstellung einer
pharmazeutischen Zusammensetzung nach Anspruch 1, dadurch **gekennzeichnet,** daß die Zusammensetzung ein nasales Spray ist.

**10.** Verfahren zur Herstellung einer
Zusammensetzung nach Anspruch 1, dadurch **gekennzeichnet,** daß (I) eine hinsichtlich der körpereigenen Weise therapautisch wirksame Menge, bedingt durch die pharmakologische Wirkung des in Salzform vorliegenden Dextrorphans, ist.

**Revendications**
**Revendications pour les Etats contractants suivants : BE, CH, DE, FR, GB, LI, LU, NL, SE**

**1.** Composition pharmaceutiquement acceptable, apte à réaliser une réponse thérapeutique systémique antitussive chez des animaux à sang chaud, comprenant par dose unitaire : (I) une quantité thérapeutiquement efficace de dextrorphane salifié, à une concentration comprise entre 10% et 15%, et (II) un

excipient non toxique, pharmaceutiquement acceptable, pour administration intranasale.

2. Composition pharmaceutique selon la revendication 1, caractérisée en ce qu'elle se présente sous la forme d'une suspension pour administration nasale.

3. Composition pharmaceutique selon la revendication 1, caractérisée en ce qu'elle se présente sous la forme d'une pommade pour administration nasale.

4. Composition pharmaceutique selon la revendication 1, caractérisée en ce qu'elle se présente sous la forme d'une crème pour administration nasale.

5. Composition pharmaceutique selon la revendication 1, caractérisée en ce qu'elle se présente sous la forme d'une solution pour administration nasale.

6. Composition pharmaceutique selon la revendication 1, caractérisée en ce qu'elle se présente sous la forme d'un gel pour administration nasale.

7. Composition pharmaceutique selon la revendication 1, caractérisée en ce qu'elle se présente sous la forme d'un gel à libération prolongée.

8. Composition pharmaceutique selon la revendication 1, caractérisée en ce qu'elle se présente sous la forme de gouttes pour administration nasale.

9. Composition pharmaceutique selon la revendication 1, caractérisée en ce qu'elle se présente sous la forme d'une pulvérisation pour administration nasale.

10. Composition selon la revendication 1, dans laquelle (I) représente une quantité thérapeutiquement efficace par voie systémique, par suite de l'action pharmacologique du dextrorphane salifié.

**Revendications pour l'Etat contractant suivant : AT**

1. Procédé préparation d'une composition pharmaceutiquement acceptable, apte à réaliser une réponse thérapeutique systémique antitussive chez des animaux à sang chaud, caractérisé eu ce que l'on mélange, par dose unitaire, (I) une quantité thérapeutiquement efficace de dextrorphane salifié, à une concentration comprise entre 10% et 15%, et (II) un excipient non toxique, pharmaceutiquement acceptable, pour administration intranasale.

2. Procédé de préparation d'une composition pharmaceutique selon la revendication 1, caractérisé en ce que la composition se présente sous la forme d'une suspension pour administration nasale.

3. Procédé de préparation d'une composition pharmacentique selon la revendication 1, caractérisé en ce que la composition se présente sous la forme d'une pommade pour administration nasale.

4. Procédé de préparation d'une composition pharmaceutique selon la revendication 1, caractérisé en ce que la composition se présente sous la forme d'une créme pour administration nasale.

5. Procédé de prépatation d'une composition pharmaceutique selon la revendication 1, caratérisé en ce que la composition se présente sous la forme d'une solution pour administration nasale.

6. Procédé de préparation d'une composition pharmaceutique selon la revendication 1, caractérisé en ce que la composition se présente sous la forme d'un gel pour administration nasale.

7. Procédé de préparation d'une composition pharmaceutique selon la revendication 1, caractérisé en ce que la composition se présente sous la forme d'nue gel à libération prolongée.

8. Procédé de préparation d'une composition pharmaceutique selon la revendication 1, caractérisé en ce que la composition se présente sous la forme de gouttes pour administration nasale.

9. Procédé de préparation d'une composition pharmaceutique selon la revendication 1, caractérisé en ce que la composition se présente sous la forme d'une pulvérisation pour administration nasale.

10. Procédé de préparation d'une composition selon la revendication 1, dans lequel (I) représente une quantité thérapeutiquement efficace par voie systémique, par suite de l'action pharmacologique du dextrorphane salifié.